(19) Europäisches Patentamt
European Patent Office
Office européen des brevets

(11) Veröffentlichungsnummer: 0 464 511 A2

# (12) EUROPÄISCHE PATENTANMELDUNG

(21) Anmeldenummer: **91110245.7**

(51) Int. Cl.5: **C07D 473/00**, C07C 43/315

(22) Anmeldetag: **21.06.91**

Ein Antrag gemäss Regel 88 EPÜ auf Hinzufügung der fehlenden Seite 10 liegt vor. Über diesen Antrag wird im Laufe des Verfahrens von der Prüfungsabteilung eine Entscheidung getroffen werden (Richtlinien für die Prüfung im EPA, A-V, 2.2).

(30) Priorität: **27.06.90 DE 4020481**

(43) Veröffentlichungstag der Anmeldung: **08.01.92 Patentblatt 92/02**

(84) Benannte Vertragsstaaten: **AT BE CH DE DK ES FR GB GR IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Jähne, Gerhard, Dr.**
**Johannesallee 14**
**W-6230 Frankfurt am Main(DE)**

(54) **Verfahren zur Herstellung von substituierten acyclischen Nukleosiden und dabei auftretende Zwischenprodukfe.**

(57) Verfahren zur Herstellung von substituierten acyclischen Nukleosiden und dabei auftretendende Zwischenprodukte
Unter Benutzung symmetrischer Formaldehydacetale der Formel

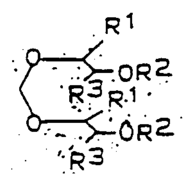

in der die Substituenten $R^1$-$R^3$ die genannten Bedeutungen haben, läßt sich vorteilhaft der Substituent

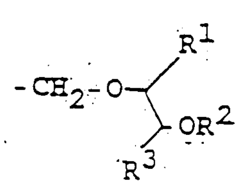

in stickstoffhaltige heterocyclische Systeme unter Bildung von acyclischer Nukleosidanaloga einführen.

EP 0 464 511 A2

Die vorliegende Erfindung betrifft ein Verfahren zu Herstellung von substituierten acyclischen Nukleosiden und dabei auftretende Zwischenprodukte. Acyclische Purinnukleoside wie 2-Amino-9-[(1,3-bis-isopropoxy-2-propoxy)methyl]-purin (EP A 0217207), wie 2-Amino-7-[(1,3-bis-isopropoxy-2-propoxy)methyl]-purin und 2-Amino-7-[(1,3-dihydroxy-2-propoxy)methyl]-purin (DE P 4008858.8), wie 9-1(1,3-Dihydroxy-2-propoxy)methyl]-guanin (EP A 0049072), wie 9-[(2,3-Dihydroxy-1-propoxy)methyl]-guanin (W.T.Ashton et al., Biochem. Biophys. Res.Commun. 108, 1716 (1982)) und wie 9-[(2-Hydroxyethoxy)methyl]-guanin (H.J.Schaeffer et al., Nature 272, 583 (1978)) sind Wirkstoffe mit antiviraler Aktivität und können durch Addition verschiedenartig aktivierter Seitenketten an das Purinsystem hergestellt werden.

Als aktivierte Seitenkettenkomponente zur Einführung der

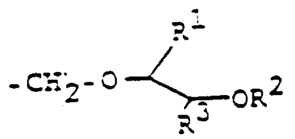 **-Substituenten**

dienen bisher die Halogenmethylether, die Methylthiomethyl-, Methylsulfinylmethyl- und Methylsulfonylmethylether und die Acyloxymethylether der entsprechend substituierten Alkanole.

Allen diesen Kupplungsmethoden sind präparative Nachteile inhärent: So sind die Halogenmethylether instabil und toxisch; die Methylthiomethyl-, Methylsulfinylmethyl- und Methylsulfonylmethylether liefern als Nebenprodukte bei der Herstellung und beim Umsatz Mercaptane, die bei der Abwasser- und Abluftreinigung Probleme bereiten, und die Acyloxymethylether sind entweder nur schwierig rein darzustellen und/oder durchlaufen bei ihrer Herstellung einen Prozeß, bei welchem Dimethylsulfoxid, inhärent mit Abwasser- und Abluftproblemen belastet, Verwendung findet.

Überraschenderweise wurde nun gefunden, daß symmetrische Formaldehydacetale bei der Herstellung von substituierten acyclischen Nukleosiden Verwendung finden können und die genannten Probleme dabei nicht auftreten.

Erfindungsgegenstand ist demzufolge ein Verfahren zu Herstellung von substituierten acyclischen Nukleosiden, dadurch gekennzeichnet, daß ein Alkohol der Formel I

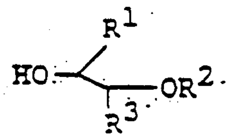 I

zu einem Formaldehydacetal der Formel II

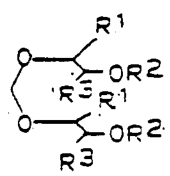 II

wobei

- $R^1$ Wasserstoff, Alkyl, gegebenenfalls einfach oder mehrfach substituiert mit Halogen, Azid, Alkoxy-, Alkenyloxy-, Alkinyloxy-, Alkylthio-, Alkenylthio-, Alkinylthio-, Dialkylamino-, Dialkenylamino-, Dialkinylamino-, Benzyloxy-, Benzylthio-, Dibenzylamino- oder Phthalimidogruppen und/oder mit $-P(O)(OR^4)(OR^5)-$, $-P(R^6)(O)(OR^5)-$, $-O-CH_2-P(O)(OR^4)(OR^5)-$ oder $-O-CH_2-P(R^6)(O)(OR^5)$-Resten, wobei $R^4$, $R^5$ und $R^6$ jeweils unabhängig voneinander Alkyl sein können, bedeutet;
- $R^2$ Alkyl, Benzyl oder ein $-CH_2-P(O)(OR^4)(OR^5)-$ oder $-CH_2-P(R^6)(O)(OR^5)$-Rest, wobei $R^4$, $R^5$ und $R^6$ jeweils unabhängig voneinander Alkyl sein können, bedeutet;
- $R^3$ Wasserstoff, Alkyl, gegebenenfalls einfach oder mehrfach substituiert mit Halogen, Azid, Alkoxy-, Alkenyloxy-, Alkinyloxy, Alkylthio-, Alkenylthio-, Alkinylthio-, Dialkylamino-, Dialkenylamino-, Dialkinylamino-, Benzyloxy-, Benzylthio-, Dibenzylamino- oder Phthalimidogruppen und/oder mit $-P(O)(OR^4)(OR^5)-$, $-P(R^6)(O)(OR^5)-$, $-O-CH_2-P(O)(OR^4)(OR^5)-$ oder $-O-CH_2-P(R^6)(O)(OR^5)$-Resten, wobei $R^4$, $R^5$ und $R^6$ jeweils unabhängig voneinander Alkyl sein können, bedeutet; umgesetzt

wird und zur Einführung der Gruppe

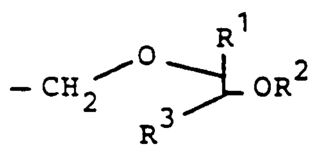

ein geeignet substituiertes stickstoffhaltiges heterocyclisches System mit einer Verbindung der Formel II zur Reaktion gebracht wird.

Das erfindungsgemäße Verfahren ist geeignet zur Herstellung von substituierten, stickstoffhaltigen Heterocyclen. Als Beispiele dafür seien angeführt:
Purine wie Purin, Adenin, 2-Chlor-6-aminopurin, Hypoxanthin, 6-Thiopurin, Xanthin, Guanin, 2-Amino-6-mercaptopurin, 2,6-Diaminopurin, 2-Aminopurin, 2,6-Dihalogenpurin; Azapurine wie 8-Azapurin, 8-Azaadenin, 8-Azaguanin; Deazapurine wie 1-Deazapurine, 3-Deazapurine, 7-Deazapurine, 9-Deazapurine; Benzimidazole; Indole; Pyrimidine wie Cytosin, 5-Halogencytosine, 4-Amino-2-mercaptopyrimidin, Uracil, 5-Halogenuracile, 4-Hydroxy-2-mercaptopyrimidin, Thymin, 4-Hydroxy-2-mercapto-5-methyl-pyrimidin, 5-(2-Bromvinyl)-uracil, 6-substituierte Pyrimidine wie 6-Phenyl-thiothymin; 2-Hydroxypyridine, 4-Hydroxypyridine; 1,2,3-Triazole, 1,2,4-Triazole, Tetrazole; Imidazole; Pyrrole.

Besondere Bedeutung besitzt es zur Herstellung von substituierten acyclischen Purin- und Pyrimidinnukleosiden.

Ganz besondere Bedeutung besitzt das erfindungsgemäße Verfahren zur Herstellung von Purinnukleosiden, wobei das eingesetzte Purinderivat eine Verbindung der Formel VI ist

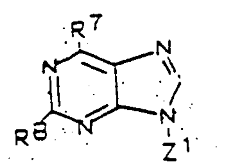

VI ,

in der $Z^1$ Trialkylsilyl, $R^7$ Halogen und $R^8$ trialkylsilyliertes Acylamino bedeuten und wobei die Reaktion zu einer Verbindung der Formel VIa führt

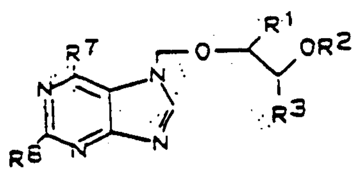

VIa ,

in der die Substituenten $R^1$-$R^8$ die obengenannten Bedeutungen haben, und anschließend der Substituent $R^8$ zur Acylamino-Gruppe solvolysiert werden kann.

Weiterhin ganz besondere Bedeutung besitzt das erfindungsgemäße Verfahren zur Herstellung von Purinnukleosiden, wobei das eingesetzte Purinderivat eine Verbindung der Formel VII ist

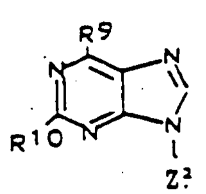

VII

in der $Z^2$ Acyl, $R^9$ Halogen und $R^{10}$ Acylamino bedeuten und wobei die Reaktion zu einer Verbindung der Formel VIIa führt,

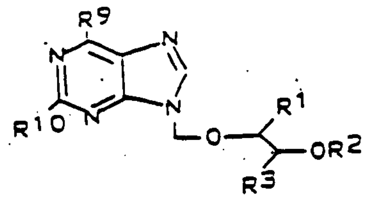

VIIa

in der die Substituenten $R^1$-$R^{10}$ die genannten Bedeutungen haben und in der durch Hydrogenolyse der Substituent $R^9$ in Wasserstoff überführt werden kann und/oder der Substituent $R^{10}$ durch Ammonolyse, Aminolyse oder Hydrolyse in $NH_2$ umgewandelt werden kann. In dem beschriebenen Verfahren sind die Substituenten $R^1$-$R^3$ bevorzugt, die nachstehend für die Verbindungen der Formel II als bevorzugt ausgewiesen werden.

Weiterhin gehören zum Gegenstand der vorliegenden Erfindung Verbindungen der Formel II,

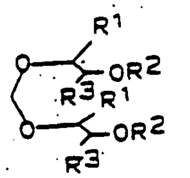

II

in der die Substituenten $R^1$-$R^3$ die obengenannten Bedeutungen haben. Besonders bevorzugt sind die Verbindungen der Formel II in denen A)

$R^1$ Wasserstoff, $C_1$-$C_6$-Alkyl, gegebenenfalls bis zu zweifach substituiert mit Halogen, Azid, $C_1$-$C_6$-Alkoxy, $C_2$-$C_6$-Alkenyloxy-, $C_2$-$C_6$-Alkinyloxy-, $C_1$-$C_6$-Alkylthio, $C_2$-$C_6$-Alkenylthio-, $C_2$-$C_6$-Alkinylthio-, $C_2$-$C_{12}$-Dialkylamino-, $C_2$-$C_{12}$-Dialkenylamino-, $C_2$-$C_{12}$-Dialkylinylamino-, Benzyloxy-, Benzylthio-, Dibenzylamino- oder Phthalimidogruppen und/oder mit $-P(O)(OR^4)(OR^5)$-, $-P(R^6)(O)(OR^5)$-, $-O-CH_2-P(O)(OR^4)(OR^5)$- oder $-O-CH_2-P(R^6)(O)(OR^5)$-Resten, wobei $R^4$, $R^5$ und $R^6$ jeweils unabhängig voneinander $C_1$-$C_6$-Alkyl sein können, bedeuten und

$R^2$ $C_1$-$C_6$-Alkyl-, $C_2$-$C_6$-Alkenyl, $C_2$-$C_6$-Alkinyl, Benzyl- oder ein $-CH_2-P(O)(OR^4)(OR^5)$- oder $-CH_2-P(R^6)(O)(OR^5)$-Rest, wobei $R^4$, $R^5$ und $R^6$ jeweils unabhängig voneinander $C_1$-$C_6$-Alkyl sein können, bedeuten und

$R^3$ Wasserstoff, $C_1$-$C_6$-Alkyl, gegebenenfalls bis zu zweifach substituiert mit Halogen, Azid, $C_1$-$C_6$-Alkoxy-, $C_1$-$C_6$-Alkenyloxy-, $C_2$-$C_6$-Alkinyloxy-, $C_1$-$C_6$-Alkylthio-, $C_2$-$C_6$-Alkenylthio-, $C_2$-$C_6$-Alkinylthio-, $C_2$-$C_{12}$-Dialkylamino-, $C_2$-$C_{12}$-Dialkenylamino-, $C_2$-$C_{12}$-Dialkinylamino-, Benzyloxy-, Benzylthio-, Dibenzylamino- oder Phthalimidogruppen und/oder mit $-P(O)(OR^4)(OR^5)$-, - $P(R^6)(O)(OR^5)$-, $-O-CH_2-P(O)(OR^4)(OR^5)$- oder $-O-CH_2-P(R^6)(O)(OR^5)$-Resten, wobei $R^4$, $R^5$ und $R^6$ jeweils unabhängig voneinander $C_1$-$C_6$-Alkyl sein können, bedeuten.

Ganz besonders bevorzugt sind Verbindungen der Formel II, in denen B)

$R^1$ Wasserstoff, $C_1$-$C_6$-Alkyl, gegebenenfalls substituiert mit $C_1$-$C_6$-Alkoxy-, $C_2$-$C_6$-Alkenyloxy-, $C_2$-$C_6$-Alkinyloxy-, $C_1$-$C_6$-Alkylthio-, Benzyloxy-, Benzylthio-, Dibenzylamino- oder Phthalimidogruppen und/oder mit $-P(O)(OR^4)OR^5)$-, - $P(R^6)(O)(OR^5)$-, $O-CH_2-P(O)(OR^4)(OR^5)$- oder $-O-CH_2-P(R^6)(O)(OR^5)$-Resten, wobei $R^4$, $R^5$ und $R^6$ jeweils unabhängig voneinander $C_1$-$C_6$-Alkyl sein können, bedeuten und

$R^2$ $C_1$-$C_6$-Alkyl, $C_2$-$C_6$-Alkenyl, $C_2$-$C_6$-Alkinyl, Benzyl oder ein $-CH_2-P(O)(OR^4)(OR^5)$-Rest, wobei $R^4$ und $R^5$ jeweils unabhängig voneinander $C_1$-$C_6$-Alkyl sein können, bedeuten und

$R^3$ Wasserstoff, $C_1$-$C_6$-Alkyl, gegebenenfalls substituiert mit $C_1$-$C_6$-Alkoxy-, $C_2$-$C_6$-Alkenyloxy-, $C_2$-$C_6$-Alkinyloxy-, $C_1$-$C_6$-Alkylthio-, Benzyloxy-, Benzylthio-, Dibenzylamino- oder Phthalimidogruppen und/oder mit $-P(O)(OR^4)(OR^5)$-, - $P(R^6)(O)(OR^5)$-, $-O-CH_2-P(O)(OR^4)(OR^5)$- oder $-O-CH_2-P(R^6)(O)(OR^5)$-Resten, wobei $R^4$, $R^5$ und $R^6$ jeweils unabhängig voneinander $C_1$-$C_6$-Alkyl sein können, bedeuten,

insbesondere Verbindungen der Formel II in denen C)

$R^1$ Wasserstoff, $C_1$-$C_6$-Alkoxymethyl, $C_1$-$C_6$-Alkenyloxymethyl, $C_1$-$C_6$-Alkinyloxymethyl, Benzyloxymethyl, $-CH_2-P(O)(OR^4)(OR^5)$, $-CH_2-CH_2-P(O)(OR^4)(OR^5)$,
$-CH_2-P(R^6)(O)(OR^5)$, $-CH_2-CH_2-P(R^6)(O)(OR^5)$, $-CH_2-O-CH_2-P(O)(OR^4)(OR^5)$ oder $-CH_2-O-CH_2-P(R^6)(O)(OR^5)$, wobei $R^4$, $R^5$ und $R^6$ jeweils unabhängig voneinander $C_1$-$C_6$-Alkyl sein können,

bedeuten und

$R^2$ $C_1$-$C_6$-Alkyl, $C_2$-$C_6$-Alkenyl, $C_2$-$C_6$-Alkinyl oder Benzyl bedeuten und

$R^3$ Wasserstoff, $C_1$-$C_6$-Alkoxymethyl, $C_2$-$C_6$-Alkenyloxymethyl, $C_2$-$C_6$-Alkinyloxymethyl, Benzyloxymethyl, $-CH_2-P(O)(OR^4)(OR^5)$, $-CH_2-CH_2-P(O)(OR^4)(OR^5)$, $-CH_2-P(R^6)(O)(OR^5)$, $-CH_2-CH_2-P(R^6)(O)(OR^5)$, $-CH_2-O-CH_2-P(O)(OR^4)(OR^5)$ oder $-CH_2-O-CH_2-P(R^6)(O)(OR^5)$, wobei $R^4$, $R^5$ und $R^6$ jeweils unabhängig voneinander $C_1$-$C_6$-Alkyl sein können, bedeuten.

Die als Substituenten genannten Alkyl-, Alkenyl- und Alkinylgruppen können geradkettig, verzweigt oder cyclisch sein. Geeignete Alkylgruppen sind beispielsweise Methyl, Ethyl, Propyl, Isopropyl, Cyclopentyl, Cyclohexyl; geeignete Alkenylgruppen sind beispielsweise Propenyl, 1-Isobuten-3-yl, 1-Cyclohexen-3-yl; geeignete Alkinylgruppen sind beispielsweise 1-Propin-3-yl oder 1-Butin-4-yl.

Die genannten Acylgruppen können geradkettig, verzweigt, cycloaliphatisch oder aromatisch sein. Geeignete Acylgruppen sind beispielsweise Acetyl, Propionyl, Butyryl, Isobutyryl, Valeroyl, Cyclopentanoyl, Cyclohexanoyl, Benzoyl oder 4-Methylbenzoyl.

Die Verbindungen der Formel II können ein oder mehrere chirale Zentren aufweisen. Die Verbindungen liegen in der Regel als Racemate vor; eine Herstellung bzw. Isolierung der reinen Enantiomere ist möglich. Gegenstand der Erfindung sind deshalb sowohl die reinen Enantiomeren als auch Mischungen derselben, wie z. B. das zugehörige Racemat.

Zum Gegenstand der vorliegenden Erfindung gehört darüber hinaus ein Verfahren zur Herstellung von Verbindungen der Formel II, das dadurch gekennzeichnet ist, daß Verbindungen der Formel I mit Paraformaldehyd, Formaldehyddimethylacetal oder Formaldehyddiethylacetal in Gegenwart eines Katalysators umgesetzt werden. Zur Durchführung des Verfahrens werden ein Alkanol der Formel I, Paraformaldehyd oder Formaldehyddimethylacetal oder Formaldehyddiethylacetal, bevorzugt Paraformaldehyd, mit einem Lösungsmittel wie z.B. Toluol, bevorzugt jedoch ohne ein Lösungsmittel, mit einem anorganischen Protonensäurekatalysator wie konzentrierter Schwefelsäure oder mit einem organischen Protonensäurekatalysator wie para-Toluolsulfonsäure oder einem Kationenaustauscher oder mit einem Lewis-Säure-Katalysator wie wasserfreiem Calciumchlorid oder Eisentrichlorid, vorzugsweise para-Toluolsulfonsäure, zusammengegeben und unter Druck im Autoklaven, bevorzugt jedoch bei Normaldruck, unter Rühren auf 50 bis 200 °C, vorzugsweise auf 100 °C, während 1 bis 24 Stunden, vorzugsweise während 3 - 7 Stunden, erhitzt. Das dabei gebildete Reaktionswasser kann, eventuell azeotrop, abdestilliert werden. Wahlweise kann ein wasserentziehendes Mittel wie aktiviertes Molekularsieb 4 Angström zugesetzt werden. Das günstigste Molverhältnis von Formaldehyd(äquivalent) : Alkanol liegt bei 0.2 : 1.0 bis 1.5 : 1.0, vorzugsweise bei 0.33 : 1.0.

Ähnliche Verfahren sind z. B. in Houben-Weyl, Methoden der organischen Chemie, Band VI/3, S. 203 ff, Georg Thieme Verlag, Stuttgart, 1965 beschrieben.

Das Reaktionsgemisch kann nach herkömmlichen Methoden aufgearbeitet werden. Die Aufarbeitung erfolgt z.B. derart, daß das Reaktionsgemisch im Vakuum fraktioniert destilliert wird, vorzugsweise jedoch so, daß das Reaktionsgemisch in einem Dialkylether gelöst, mehrmals mit Wasser ausgeschüttelt und über Natriumsulfat getrocknet wird, bevor es im Vakuum fraktioniert destilliert wird. Die fraktionierte Destillation liefert neben dem symmetrischen Formaldehydacetal der Formel II nicht umgesetztes Alkanol der Formel I zurück.

Weiterhin gehört zum Gegenstand der vorliegenden Erfindung ein Verfahren zur Herstellung von mit einer

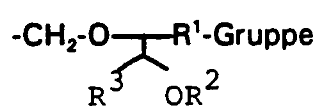

substituierten acyclischen Nukleosiden, das dadurch gekennzeichnet ist, daß eine Verbindung der Formel II mit einem geeigneten stickstoffhaltigen Heterocyclus umgesetzt wird, wobei die Substituenten $R^1$-$R^3$ oben genannten Bedeutungen haben.

Insbesondere gehört zum Gegenstand der vorliegenden Erfindung ein Verfahren zu Herstellung der obengenannten Verbindungen der Formel VIa, dadurch gekennzeichnet, daß das eingesetzte Purinderivat eine Verbindung der obengenannten Formel VI ist, in der $Z^1$ Trialkylsilyl, insbesondere Trimethylsilyl, $R^7$ Halogen, insbesondere Chlor und $R^8$ trialkylsilyliertes Acylamino, vorzugsweise trimethylsilyliertes Benzoylamino oder trimethylsilyliertes $C_1$-$C_8$ aliphatisches Acylamino, insbesondere trimethylsilyliertes Acetamino bedeuten.

Das Purinderivat wird mit einem erfindungsgemäßen symmetrischen Formaldehydacetal der Formel II, dessen Reste $R^1$, $R^2$ und $R^3$ wie oben unter A), B) oder C) beschrieben umgesetzt, vorzugsweise in einem

aprotischen Lösungsmittel wie Benzol, Toluol, Xylol, Acetonitril, Dichlormethan oder 1,2-Dichlorethan oder Gemischen davon in Gegenwart eines sauren Katalysators, bevorzugt eines Lewis-Säure-Katalysators wie Aluminiumtrichlorid, Aluminiumsulfat, Eisentrichlorid, Galliumtrichlorid, Zinntetrachlorid, Titantetrachlorid, Bortrifluorid, Cäsiumfluorid, Cäsiumsulfat oder Trifluormethansulfonsäuretrialkylsilylester, besonders Trifluormethansulfonsäuretrimethylsilylester, wobei die Mengen dieser Katalysatorreagenzien 0,1-10, vorzugsweise 0,8-7 Äquivalente, bezogen auf die Menge des jeweils eingesetzten symmetrischen Formaldehydacetals, betragen, bei Temperaturen zwischen -70°C und + 80°C, vorzugsweise zwischen -40°C und + 30°C, während 2 bis 24 Stunden, vorzugsweise während 2 bis 6 Stunden.

Dieses Verfahren liefert in hoher Regioselektivität, in der Regel $\gg$ 9:1, bevorzugt das N7-Isomer des jeweiligen Purinderivates der Formel VIa mit $R^7$ = Acylamino, nachdem die labile Trialkylsilylschutzgruppe durch milde Solvolyse mit Wasser, mit wäßrigem oder alkoholischem Ammoniak oder mit wäßriger Natriumhydrogencarbonatlösung oder durch Alkoholyse entfernt wurde.

Die Produkte der Formel VIa können, wie in der deutschen Patentanmeldung P 40 08 858.8 beschrieben, zu anderen Purinderivaten weiter umgesetzt werden.

Zum Gegenstand der vorliegenden Erfindung gehört darüber hinaus ein Verfahren zur Herstellung der obengenannten Verbindungen der Formel VIIa, das dadurch gekennzeichnet ist, daß das eingesetzte Purinderivat eine Verbindung der obengenannten Formel VII ist, in der $Z^2$ Benzoyl oder $C_1$-$C_8$ aliphatisches oder cycloaliphatisches Acyl, insbesondere Acetyl, $R^9$ Halogen, insbesondere Chlor und $R^{10}$ Benzoyl- oder $C_1$-$C_8$ aliphatisches oder cycloaliphatisches Acylamino, insbesondere Acetamino bedeuten. Das Purinderivat wird mit einem erfindungsgemäßen symmetrischen Formaldehydacetal der Formel II, dessen Reste $R^1$, $R^2$ und $R^3$ wie oben unter A), B) oder C) beschrieben umgesetzt, vorzugsweise in einem polaren aprotischen Lösungsmittel wie Sulfolan, Dimethylsulfoxid, Dimethylacetamid, Dimethylformamid oder N-Methylpyrrolidon-(2) oder Gemischen davon, vorzugsweise N-Methylpyrrolidon-(2), in Gegenwart eines protischen sauren Katalysators wie para-Toluolsulfonsäure oder Phosphorsäure- bis-(4-nitrophenylester), bevorzugt eines Lewis-Säure-Katalysators wie Aluminiumtrichlorid, Aluminiumsulfat, Eisentrichlorid, Galliumtrichlorid, Zinntetrachlorid, Titantetrachlorid, Cäsiumfluorid, Cäsiumsulfat, Bortrifluorid oder Bortrifluorid-Dialkylether-Komplex, besonders bevorzugt Aluminiumsulfat oder Bortrifluorid-Dialkylether-Komplex, wobei die Mengen diese Katalysatorreagenzien 0,1-10, vorzugsweise 0,2-5 Äquivalente, bezogen auf die Menge des jeweils eingesetzten symmetrischen Formaldehydacetals, betragen, bei Temperaturen zwischen 0°C und 200°C, vorzugsweise zwischen 70°C und 120°C, während 2-24 Stunden, vorzugsweise während 2-8 Stunden. Dieses Verfahren liefert in hoher Regioselektivität, in der Regel $\gg$ 9:1, bevorzugt das N9-Isomere des jeweiligen Purinderivates der Formel VIIa.

Weiterhin gehören zum Gegenstand der vorliegenden Patentanmeldung Verbindungen der Formel

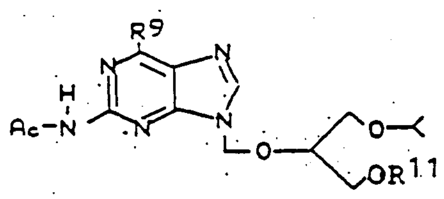

in der $R^9$ Halogen oder Wasserstoff und $R^{11}$ Isopropyl oder Benzyl bedeutet.

Diese Verbindungen haben antivirale Aktivität und sind darüber hinaus von Bedeutung bei der Herstellung von anderen Purinnukleosiden.

Die vorliegende Erfindung wird durch die nachfolgenden Ausführungsbeispiele näher erläutert.

**Beispiel 1.:**

Verbindung der Formel II, worin $R^1$ = Isopropoxymethyl, $R^2$ = Isopropoxy und $R^3$ = Wasserstoff ist: 105.6 g (0.6 Mol) 1,3-Bis-isopropoxy-propan-2-ol werden mit 6 g (0.2 Mol) Paraformaldehyd und 0.25 g para-Toluolsulfonsäure unter Rühren langsam auf 100°C erwärmt. Die Reaktionsmischung wird 4 Stunden bei dieser Temperatur gerührt. Das abgekühlte Reaktionsgemisch wird in Diethylether gelöst, mehrmals mit Wasser ausgeschüttelt und die organische Phase anschließend über Natriumsulfat getrocknet. Die fraktionierte Destillation liefert 58.5 g Ausgangsalkohol vom Siedepunkt 60°C bei 0.7 mm Hg und 46.5 g (61.5% bez. auf Paraformaldehyd) Formaldehyd-bis-(1,3-bis-isoprop-oxy-2-propyl)-acetal vom Siedepunkt 130 - 134°C bei 0.7 mm Hg.1H NMR (60 MHz, $CDCl_3$), $\delta$ [ppm]: 4.93 (s, 2H), 4.13 - 3.33 (m, 14H), 1.15 (d, 24H).

In ähnlicher Weise können hergestellt werden
Formaldehyd-bis-(1,3-bis-methoxy-2-propyl)-acetal
Formaldehyd-bis-(1,3-bis-ethoxy-2-propyl)-acetal
Formaldehyd-bis-(1,3-bis-propoxy-2-propyl)-acetal
Formaldehyd-bis-(1,3-bis-butoxy-2-propyl)-acetal
Formaldehyd-bis-(1,3-bis-cyclopentyloxy-2-propyl)-acetal
Formaldehyd-bis-(1,3-bis-cyclohexyloxy-2-propyl)-acetal
Formaldehyd-bis-[1,3-bis(butyl-2-oxy)-2-propyl]-acetal
Formaldehyd-bis-[1,3-bis(2-methyl-propyl-3-oxy)-2-propyl]-acetal
Formaldehyd-bis-(1-isopropoxy-3-methoxy-2-propyl)-acetal
Formaldehyd-bis-(1-isopropoxy-3-ethoxy-2-propyl)-acetal
Formaldehyd-bis-(1-isopropoxy-3-propoxy-2-propyl)-acetal
Formaldehyd-bis-(1-isopropoxy-3-butoxy-2-propyl)-acetal
Formaldehyd-bis-(1-isopropoxy-3-cyclopentyloxy-2-propyl)-acetal
Formaldehyd-bis-(1-isopropoxy-3-cyclohexyloxy-2-propyl)-acetal
Formaldehyd-bis-[1,3-bis(1-propenyl-3-oxy)-2-propyl]-acetal
Formaldehyd-bis-[1-isopropoxy-3-(1-propenyl-3-oxy)-2-propyl]-acetal
Formaldehyd-bis-(1,3-bis-benzyloxy-2-propyl)-acetal
Formaldehyd-bis-(1-benzyloxy-3-methoxy-2-propyl)-acetal
Formaldehyd-bis-(1-benzyloxy-3-ethoxy-2-propyl)-acetal
Formaldehyd-bis-(1-benzyloxy-3-propoxy-2propyl)-acetal
Formaldehyd-bis-(1-benzyloxy-3-isopropoxy-2-propyl)-acetal
Formaldehyd-bis-(1-isopropoxy-3-ethylthio-2-propyl)-acetal
Formaldehyd-bis-(1-benzyloxy-3-ethylthio-2-propyl)-acetal
Formaldehyd-bis-(1-isopropoxy-3-methylthio-2-propyl)-acetal
Formaldehyd-bis-(1-benzyloxy-3-methylthio-2-propyl)-acetal
Formaldehyd-bis-(1-isopropoxy-3-N-phthalimido-2-propyl)-acetal
Formaldehyd-bis-(1-benzyloxy-3-N-phthalimido-2-propyl)-acetal
Formaldehyd-bis-[1-isopropoxy-3-((O,O-di-isopropyl)phosphonylmethoxy)-2-propyl]-acetal
Formaldehyd-bis-[1-isopropoxy-4-((O,O-di-isopropyl)phosphonyl)-2-butyl]-acetal
Formaldehyd-bis-[1-isopropoxy-4-(isopropoxy(methyl)phosphoryl)-2-butyl]-acetal
Formaldehyd-bis-(1-isopropoxy-2-benzyloxy-3-propyl)-acetal
Formaldehyd-bis-(1-isopropoxy-2-methoxy-3-propyl)-acetal
Formaldehyd-bis-(1-isopropoxy-2-ethoxy-3-propyl)-acetal
Formaldehyd-bis-(1-isopropoxy-2-propoxy-3-propyl)-acetal
Formaldehyd-bis-(1,2-bis-isopropoxy-3-propyl)-acetal
Formaldehyd-bis-(1,2-bis-benzyloxy-3-propyl)-acetal
Formaldehyd-bis-(1-benzyloxy-2-methoxy-3-propyl)-acetal
Formaldehyd-bis-(1-benzyloxy-2-isopropoxy-3-propyl)-acetal
Formaldehyd-bis-(2-benzyloxy-1-ethylthio-3-propyl)-acetal
Formaldehyd-bis-(2-methoxy-1-ethylthio-3-propyl)-acetal
Formaldehyd-bis-(2-isopropoxy-1-ethylthio-3-propyl)-acetal
Formaldehyd-bis-(2-benzyloxy-1-N-phthalimido-3-propyl)-acetal
Formaldehyd-bis-[2-benzyloxy-1-((O,O-di-isopropyl)phosphonylmethoxy)-3-propyl]-acetal
Formaldehyd-bis-[1-isopropoxy-2-((O,O-di-isopropyl)phosphonylmethoxy)-3-propyl]-acetal
Formaldehyd-bis-(2-isopropoxy-1-ethyl)-acetal
Formaldehyd-bis-(2-methoxy-1-ethyl)-acetal
Formaldehyd-bis-(2-ethoxy-1-ethyl)-acetal
Formaldehyd-bis-(2-propoxy-1-ethyl)-acetal
Formaldehyd-bis-(2-butoxy-1-ethyl)-acetal
Formaldehyd-bis-(2-benzyloxy-1-ethyl)-acetal
Formaldehyd-bis-(2-ethylthio-1-ethyl)-acetal
Formaldehyd-bis-[2-((O,O-di-isopropyl)phosphonylmethoxy)-1-ethyl]-acetal
Formaldehyd-bis-[2-(isopropoxy(methyl)phosphoryl)-1-ethyl]-acetal

**Beispiel 2.1.:**

Verbindung der Formel VIa, worin $R^7$ = Chlor, $R^8$ = Acetamido, $R^1$ = Isopropoxymethyl, $R^2$ =

Isopropyl und $R^3$ = Wasserstoff ist:
6.9 g (0.033 Mol) 2-Acetamido-6-chlor-purin werden in 28 ml trockenem Xylol mit 28 ml Hexamethyldisilazan (HMDS) und 0.2 g Ammoniumsulfat 3 Stunden bei Rückflußtemperatur umgesetzt und ergeben eine Verbindung der Formel VI, worin $R^7$ Chlor, $R^8$ trimethylsilyliertes Acetamido und $Z^1$ Trimethylsilyl ist. Das Reaktionsgemisch wird vom Lösungsmittel und überschüssigen HMDS befreit, in 85 ml trockenem 1,2-Dichlorethan gelöst und bei -30°C zu einer Lösung von 11.96 g (0.033 Mol) Formaldehyd-bis-(1,3-bis-isopropoxy-2-propyl)-acetal (Verbindung aus Beispiel 1) in 85 ml trockenem 1,2-Dichlorethan gegeben. Sodann werden bei -30°C langsam 5 ml (0.026 mol) Trifluormethansulfonsäuretrimethylsilylester zugefügt und das Reaktionsgemisch 2 Stunden bei -30°C gerührt. Danach wird die Mischung in 500 ml Eiswasser eingerührt und filtriert. Der Rückstand wird mit 50 ml 1,2-Dichlorethan gewaschen. Die organische Phase wird abgetrennt, und die wäßrige Phase wird 3 x mit 100 ml 1,2-Dichlorethan ausgeschüttelt. Die vereinigten organischen Phasen werden 1 x mit 100 ml Wasser, 2 x mit 100 ml verdünnter Natriumhydrogencarbonatlösung und nochmals mit 100 ml Wasser ausgeschüttelt, über Natriumsulfat getrocknet, filtriert und eingeengt. Der Rückstand, dessen HPLC-Analyse ein Verhältnis der N7 : N9 -Substitution von 10 : 1 anzeigt, wird über Kieselgel mit Essigester/Methanol 15/1 chromatographisch gereinigt und liefert 7.64 g (58% d. Th.) 2-Acetamido-6-chlor-7-[(1,3-bis-isopropoxy-2-propoxy)methyl]-purin vom Schmelzpunkt 73 - 75°C. 1H NMR (270 MHz, $d_6$-DMSO), δ [ppm]: 10.68 (s, 1H), 8.84 (s, 1H), 5.81 (s, 2H), 3.71 (m, 1H), 3.46 - 3.24 (m, 6H), 2.18 (s, 3H), 0.90 (m, 12H).

**Beispiel 2.2.:**

Verbindung der Formel VIa, worin $R^7$ = Chlor, $R^8$ = Acetamido, $R^1$ = Wasserstoff, $R^2$ = Isopropyl und $R^3$ = Wasserstoff ist:
27.9 g (0.132 Mol) 2-Acetamido-6-chlor-purin werden wie in Beispiel 2.1. beschrieben zur Bis-trimethylsilylverbindung der Formel VI mit $R^7$ = Chlor, $R^8$ = trimethylsilyliertes Acetamido und $Z^1$ = Trimethylsilyl umgesetzt, in 340 ml 1,2-Dichlorethan gelöst und bei -30°C zu einer Lösung von 21.7 g (0.096 Mol) Formaldehyd-bis-(2-isopropoxy-1-ethyl)-acetal (Verbindung der Formel II, worin $R^1$ = Wasserstoff, $R^2$ = Isopropyl und $R^3$ = Wasserstoff ist) in 340 ml 1,2-Dichlorethan zugegeben. Unter Rühren bei -30°C werden 20 ml (0.104 Mol) Trifluormethansulfonsäuretrimethylsilylester zugetropft. Nach 2 Stunden bei -30°C wird das Reaktionsgemisch wie im Beispiel 2.1. aufgearbeitet und liefert 15.8 g (50% d. Th. bezogen auf das Formaldehydacetal der Formel II) 2-Acetamido-6-chlor-7-(2-isopropoxyethoxymethyl)-purin mit dem Schmelzpunkt 116 - 117°C. 1H NMR (270 MHz, $d_6$-DMSO), δ [ppm]: 10.51 (s, 1H), 8.86 (s, 1H), 5.78 (s, 2H), 3.60 (m, 2H), 3.50 - 3.40 (m, 3H), 2.19 (s, 3H), 0.97 (d,6H).

**Beispiel 2.3.:**

Verbindung der Formel VIa, worin $R^7$ = Chlor, $R^8$ = Acetamido, $R^1$ = Wasserstoff, $R^2$ = Benzyl und $R^3$ = Isopropoxymethyl ist:
Die analoge Umsetzung mit Formaldehyd-di-(2-benzyloxy-3-isopropoxy-1-propyl)-acetal (Verbindung der Formel II, worin $R^1$ = Wasserstoff, $R^2$ = Benzyl und $R^3$ = Isopropoxymethyl ist) liefert in 51 %iger Ausbeute 2-Acetamido-6-chlor-7-[(2-benzyloxy-3-isopropoxy-1-propoxy)methyl]-purin als gelblichen Sirup. 1H NMR (60 MHz, $d_6$-DMSO), δ [ppm]: 10.73 (s, 1H), 8.90 (s, 1H), 7.28 (s, 5H), 5.80 (s, 2H), 4.52 (s, 2H), 3.65 - 3.20 (m, 6H), 2.20 (s, 3H), 0.92 (d, 6H).

**Beispiel 3.1.:**

Verbindung der Formel VIIa, worin $R^9$ = Chlor, $R^{10}$ = Acetamido, $R^1$ = Isopropoxymethyl, $R^2$ = Isopropyl und $R^3$ = Wasserstoff ist:
Zu einer Lösung von 2.54 g (0.01 Mol) $N^2$,$N^9$-Diacetyl-6-chlor-purin (Verbindung der Formel VII, worin $R^9$ Chlor, $R^{10}$ Acetamido und $Z^2$ Acetyl ist) in 25 ml trockenem N-Methyl-pyrrolidon-(2) (NMP) werden 4.5 g (0.011 Mol) Formaldehyd- bis-(1,3-bis-isopropoxy-2-propyl)-acetyl (Verbindung der Formel II, worin $R^1$ Isopropoxymethyl, $R^2$ Isopropoxy und $R^3$ Wasserstoff ist) zugefügt. Unter Rühren tropft man 2.84 g (2.5 ml, 0.01 Mol) einer 50%igen Bortrifluoridetheratlösung zu und rührt dann 4 Stunden bei 100°C.
Die abgekühlte Reaktionsmischung wird in ein Gemisch aus Eis, Wasser und Dichlormethan eingerührt, die organische Phase wird abgetrennt und die wäßrige Phase wird 3 x mit Dichlormethan extrahiert, worauf die vereinigten organischen Phasen mit Wasser ausgeschüttelt, über Natriumsulfat getrocknet und vom Lösungsmittel befreit werden. Die HPLC-Analyse des Rohproduktes ergibt ein Isomerenverhältnis von N9 : N7 wie 85 : 1.7. Verrühren des Rohproduktes mit Diisopropylether liefert 2.5 g (62.6% d. Th.)

2-Acetamido-6-chlor-9-[(1,3-bis-isopropoxy-2-propoxy)methyl]-purin vom Schmelzpunkt 106° C. 1H NMR (60 MHz, $d_6$-DMSO), δ [ppm]:10.83 (s, 1H), 8.65 (s, 1H), 5.70 (s, 2H), 4.03 - 3.67 (m, 1H), 3.63 - 3.17 (m, 6H), 2.22 (s, 3H), 0.90 (d, 12H).

**Beispiel 3.2.:**

Die Verbindung des Beispiels 3.1. läßt sich auch gewinnen, wenn man 0.01 Mol $N^2$,$N^9$-Diacetyl-6-chlor-purin mit 0.011 Mol Formaldehyd-bis-(1,3-bis-isopropoxy-2-propyl)-acetal und 0.1 Mol wasserfreiem Aluminiumsulfat in trockenem NMP sechs Stunden bei 100° C behandelt. Die HPLC-Analyse des Rohproduktes gibt ein Isomerenverhältnis von N9- : N7-Isomer wie 73.6 : 8.2 an. 2-Acetamido-6-chlor-9-[(1,3-bis-isopropoxy-2-propoxy)methyl]-purin wird in 61%iger Ausbeute isoliert.

**Beispiel 3.3.:**

Des weiteren läßt sich die Verbindung des Beispiels 3.1. gewinnen, wenn man 0.01 Mol $N^2$,$N^9$-Diacetyl-6-chlor-purin mit 0.011 Mol Formaldehyd-bis-(1,3-bis-isopropoxy-2-propyl)-acetal in wasserfreiem Dimethylformamid mit 0.05 Mol Zinntetrachlorid acht Stunden bei 100° C rührt. Die HPLC-Analyse des Rohproduktes zeigt ein Isomerenverhältnis von N9 : N7 wie 93.2 : 2.4 an. Die Ausbeute an 2-Acetamido-6-chlor-9-[(1,3-bis-isopropoxy-2-propoxy)methyl]-purin beträgt 64%.

**Beispiel 3.4.:**

Die Durchführung der Reaktion wie in Beispiel 3.3., jedoch mit NMP anstelle von Dimethylformamid als Lösungsmittel, liefert laut HPLC-Analyse 95.5% N9-Isomer und 2.4% N7-Isomer. Die Ausbeute an 2-Acetamido-6-chlor-9-[(1,3-bis-isopropoxy-2-propoxy)methyl]-purin beträgt 75%.

**Beispiel 3.5.:**

Verbindung der Formel VIIa, worin $R^9$ = Chlor, $R^{10}$ = Acetamido, $R^1$ = Wasserstoff, $R^2$ = Isopropyl und $R^3$ = Wasserstoff ist:
Der Umsatz von 12.7 g (0.05 Mol) $N^2$,$N^9$-Diacetyl-6-chlor-purin mit 22.5 g (0.55 Mol) Formaldehyd-bis-(2-isopropoxy-1-ethyl)-acetal (Verbindung der Formel II, worin $R^1$ Wasserstoff, $R^2$ Isopropyl und $R^3$ Wasserstoff ist) und 65 g (0.25 Mol) Zinntetrachlorid in 125 ml NMP liefert nach 4 Stunden bei 100° C nach Aufarbeitung wie oben beschrieben und nach chromatographischer Reinigung an Kieselgel mit Essigester/Methanol 9/1 9.5 g (58% d. Th.) 2-Acetamido-6-chlor-9-[(2-isopropoxy-ethoxy)methyl]-purin vom Schmelzpunkt 135 - 137° C. 1H NMR (270 MHz, $d_6$-DMSO), δ [ppm]: 10.84 (s, 1H), 8.64 (s, 1H), 5.60 (s, 2H), 3.69 - 3.65 (m, 2H), 3.54 - 3.41 (m, 3H), 2.21 (s, 3H), 0.99 (d, 6H).

**Beispiel 3.6.:**

Verbindung der Formel VIIa, worin $R^9$ = Chlor, $R^{10}$ = Acetamido, $R^1$ = Wasserstoff, $R^2$ = Benzyl und $R^3$ = Isopropoxymethyl ist:
Der dem Beispiel 3.5. analoge Umsatz mit Formaldehyd-bis-(2-benzyloxy-3-isopropoxy-1-propyl)-acetal (Verbindung der Formel II, worin $R^1$ Wasserstoff, $R^2$ Benzyl und $R^3$ Isopropoxy ist) liefert in 53 %iger Ausbeute 2-Acetamido-6-chlor-9-[(2-benzyloxy-3-isopropoxy-1-propoxy)methyl]-purin vom Schmelzpunkt 87 - 89° C.

1H NMR (60 MHz, $d_6$-DMSO), δ [ppm]: 10.87 (s, 1H), 8.67 (s, 1H), 7.28 (s, 5H), 5.63 (s, 2H), 4.51 (s, 2H), 3.73 - 3.22 (m, 6H), 2.20 (s, 3H), 0.95 (d, 6H).

**Beispiel 3.7.:**

Verbindung der Formel VIIa, worin $R^9$ = Chlor, $R^{10}$ = Acetamido, $R^1$ = Benzyloxymethyl, $R^2$ = Isopropyl und $R^3$ = Wasserstoff ist:
Der dem Beispiel 3.1. analoge Umsatz mit Formaldehyd-bis-(1-benzyloxy-3-isopropoxy-2-propyl)-acetal (Verbindung der Formel II, worin $R^1$ Benzyloxymethyl, $R^2$ Isopropyl und $R^3$ Wasserstoff ist) liefert 2-Acetamido-6-chlor-9-[(1-benzyloxy-3-isopropoxy-2-propoxy)methyl]purin. Öl, 1H NMR (270 MHz, $d_6$-DMSO), δ [ppm]:10.82 (s, 1H), 8.63 (s, 1H), 7.33 - 7.14 (m, 5H), 5.69 (s, 2H), 4.39 (s, 2H), 4.04 (m, 1H), 3.49 - 3.25 (m, 5H), 2.20 (s, 3H), 0.91 (2d, 6H).

Verbindungen der Formel VIIa, worin $R^9$ Halogen, bevorzugt Chlor, $R^{10}$ Benzoyl- oder $C_1$ - $C_8$ aliphatisches oder cycloaliphatisches Acylamino, bevorzugt Acetamido, und $R^1$, $R^2$ und $R^3$ wie oben definiert ist, können in Verbindungen der Formel VIIa, worin $R^9$ Wasserstoff, $R^{10}$ Benzoyl- oder $C_1$ - $C_8$ aliphatisches oder cyclo-aliphatisches Acylamino, bevorzugt Acetamino, oder Amino und $R^1$ bevorzugt Isopropoxymethyl, $R^2$ bevorzugt Isopropyl und $R^3$ bevorzugt Wasserstoff ist, umgewandelt werden. Das bevorzugte Verfahren beinhaltet die Hydrogenolyse der Gruppe $R^9$ und die Ammonolyse, Aminolyseoder Hydrolyse der Gruppe $R^{10}$ von Verbindungen der Formel VIIa, worin $R^1$, $R^2$ und $R^3$ wie oben definiert sind.

**Beispiel 4.1.:**

Umwandlung einer Verbindung der Formel VIIa, worin $R^9$ = Chlor, $R^{10}$ = Acetamido, $R^1$ = Isopropoxymethyl, $R^2$ = Isopropoxy und $R^3$ = Wasserstoff ist, in eine Verbindung der Formel VIIa, worin $R^9$ = Wasserstoff, $R^{10}$ = Acetamido, $R^1$ = Isopropoxymethyl, $R^2$ = Isopropyl und $R^3$ = Wasserstoff ist:
10.5 g (0.026 Mol) der Verbindung des Beispiels 3.1. werden in 220 ml Methanol gelöst, mit 2.2 g Palladium auf Kohle (10 %) und mit 7 ml Triethylamin versetzt und bei Raumtemperatur mit Wasserstoff behandelt. Nachdem die theoretische Menge Wasserstoff aufgenommen ist, wird vom Katalysator abfiltriert, der Rückstand mit Methanol gewaschen und die Lösung vom Lösungsmittel befreit. Der kristalline Rückstand wird in Essigester angerührt, filtriert und der Rückstand mit Essigester gewaschen. Das Filtrat wird vom Lösungsmittel befreit und chromatographisch über Kieselgel mit Essigester/Methanol 9/1 gereinigt. Man erhält 8.9 g (93.8 % d. Th.) 2-Acetamido-9-[(1,3-bis-isopropoxy-2-propoxy)methyl]-purin vom Schmelzpunkt 85 - 86 °C. 1H NMR (60 MHz, $d_6$-DMSO), $\delta$ [ppm]: 10.60 (s, 1H), 9.03 (s, 1H), 8.60 (s, 1H), 5.70 (s, 2H), 3.93 (m, 1H), 3.67 - 3.22 (m, 6H), 2.25 (s, 3H), 0.93 (d, 12H).

**Beispiel 4.2.:**

Verbindung der Formel VIIa, worin $R^9$ = Wasserstoff, $R^{10}$ = Acetamido, $R^1$ = Benzyloxymethyl, $R^2$ = Isopropyl und $R^3$ Wasserstoff ist:
In analoger Weise wie in Beispiel 4.1. beschrieben läßt sich aus der Verbindung des Beispiels 3.7. 2-Acetamido-9-[(1-benzyloxy-3-isopropoxy-2-propoxy)methyl]purin gewinnen. Öl, 1H NMR (270 MHz, $d_6$-DMSO), $\delta$ [ppm]:10.58 (s, 1H), 8.98 (s, 1H), 8.57 (s, 1H), 7.34 - 7.17 (m, 5H), 5.69 (s, 2H), 4.39 (s, 2H), 4.03 (m, 1H), 3.49 - 3.25 (m, 5H), 2.21 (s, 3H), 0.92 (d, 6H).

**Beispiel 4.3.:**

Umwandlung einer Verbindung der Formel VIIa, worin $R^9$ = Wasserstoff, $R^{10}$ = Acetamido, $R^1$ = Isopropoxymethyl, $R^2$ = Isopropyl und $R^3$ = Wasserstoff ist, in eine Verbindung der Formel VIIa, worin $R^9$ = Wasserstoff, $R^{10}$ = Amino, $R^1$ = Isopropoxymethyl, $R^2$ = Isopropyl und $R^3$ = Wasserstoff ist:
17 g (0.047 Mol) der Verbindung des Beispiels 4.1. werden in 85 ml Methanol und 85 ml 40 %iger wäßriger Methylaminlösung zwei Stunden unter Rückfluß erhitzt. Die abgekühlte Lösung wird vom Methanol befreit, mit Aktivkohle behandelt, filtriert, mit verdünnter Essigsäure neutralisiert, mit Natriumchlorid gesättigt und mehrmals mit Dichlormethan ausgeschüttelt. Die organische Phase wird mit gesättigter Kochsalzlösung gewaschen, über Natriumsulfat getrocknet, vom Lösungsmittel befreit und chromatographisch über Kieselgel mit Essigester/Methanol 9/1 gereinigt. Auf diese Weise werden 13.2 g (86.9 % d. Th.) 2-Amino-9-[(1,3-diisopropoxy-2-propoxy)methyl]-purin vom Schmelzpunkt 89 - 90 °C erhalten.
1H NMR (270 MHz, $d_6$-DMSO), $\delta$ [ppm]: 8.59 (s, 1H), 8.16 (s, 1H), 6.52 (s, 2H), 5.52 (s, 2H), 3.80 (m, 1H), 3.49 - 3.21 (m, 6H), 0.97 (m, 12H).

**Beispiel 4.4.:**

Verbindung der Formel VIIa, worin $R^9$ = Wasserstoff, $R^{10}$ = Amino, $R^1$ = Benzyloxymethyl, $R^2$ = Isopropyl und $R^3$ = Wasserstoff ist:
In analoger Weise wie in Beispiel 4.3. beschrieben läßt sich aus der Verbindung des Beispiels 4.2. 2-Amino-9-[(1-benzyloxy-3-isopropoxy-2-propoxy)methyl]purin gewinnen.

**Patentansprüche**

1. Verfahren zur Herstellung von substituierten acyclischen Nukleosiden, dadurch gekennzeichnet, daß ein Alkohol der Formel I

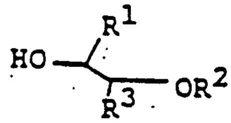

I

zu einem Formaldehydacetal der Formel II

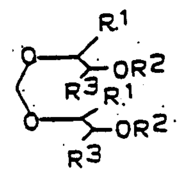

II

wobei

$R^1$ Wasserstoff, Alkyl, gegebenenfalls einfach oder mehrfach substituiert mit Halogen, Azid, Alkoxy-, Alkenyloxy-, Alkinyloxy-, Alkylthio-, Alkenylthio-, Alkinylthio-, Dialkylamino-, Dialkenylamino-, Dialkinylamino-, Benzyloxy-, Benzylthio-, Dibenzylamino-, oder Phthalimidogruppen und/oder mit - $P(O)(OR^4)(OR^5)$-, $-P(R^6)(O)(OR^5)$-, $-O-CH_2-P(O)(OR^4)(OR^5)$- oder $-O-CH_2P(R^6)(O)(OR^5)$-Resten, wobei $R^4$, $R^5$ und $R^6$ jeweils unabhängig voneinander $C_1$-$C_6$-Alkyl sein können, bedeutet;

$R^2$ Alkyl, Benzyl oder ein $-CH_2-P(O)(OR^4)(OR^5)$- oder $-CH_2-P(R^6)(O)(OR^5)$-Rest, wobei $R^4$, $R^5$ und $R^6$ jeweils unabhängig voneinander $C_1$-$C_6$-Alkyl sein können, bedeutet

$R^3$ Wasserstoff, Alkyl, gegebenenfalls einfach oder mehrfach substituiert mit Halogen, Azid, Alkoxy-, Alkenyloxy-, Alkinyloxy-, Alkylthio-, Alkenylthio-, Alkinylthio-, Dialkylamino-, Dialkenylamino-, Dialkinylamino-, Benzyloxy-, Benzylthio-, Dibenzylamino- oder Phthalimidogruppen und/oder mit $-P(O)(OR^4)(OR^5)$-, $-P(R^6)(O)(OR^5)$-, $-O-CH_2-P(O)(OR^4)(OR^5)$- oder $-O-CH_2-P(R^6)(O)(OR^5)$-Resten,
wobei $R^4$, $R^5$ und $R^6$ jeweils unabhängig voneinander $C_1$-$C_6$-Alkyl sein können, bedeutet, umgesetzt wird und zur Einführung der Gruppe

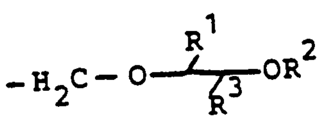

ein geeignet substituiertes stickstoffhaltiges heterocyclisches System mit einer Verbindung der Formel II zur Reaktion gebracht wird.

2. Verfahren gemäß Anspruch 1, dadurch gekennzeichnet, daß das stickstoffhaltige heterocyclische System ausgewählt ist aus der folgenden Gruppe von Verbindungen:

Purine wie Purin, Adenin, 2-Chlor-6-aminopurin, Hypoxanthin, 6-Thiopurin, Xanthin, Guanin, 2-Amino-6-mercaptopurin, 2,6-Diaminopurin, 2-Aminopurin, 2,6-Dihalogenpurin; Azapurine wie 8-Azapurin, 8-Azaadenin, 8-Azaguanin; Deazapurine wie 1-Deazapurine, 3-Deazapurine, 7-Deazapurine, 9-Deazapurine; Benzimidazole; Indole; Pyrimidine wie Cytosin, 5-Halogencytosine; 4-Amino-2-mercaptopyrimidin, Uracil, 5-Halogenuracile, 4-Hydroxy-2-mercaptopyrimidin, Thymin, 4-Hydroxy-2-mercapto-5-methyl-pyrimidin, 5-(2-Bromvinyl)uracil, 6-substituierte Pyrimidine wie 6-Phenylthiothymin; 2-Hydroxypyridine, 4-Hydroxypyridine; 1,2,3-Triazole, 1,2,4-Triazole, Tetrazole; Imidazole; Pyrrole.

3. Verfahren gemäß den Ansprüchen 1 oder 2, dadurch gekennzeichnet, daß das stickstoffhaltige heterocyclische System ein Purinderivat ist.

4. Verfahren gemäß einem oder mehreren der Ansprüche 1-3, dadurch gekennzeichnet, daß das eingesetzte Purinderivat eine Verbindung der Formel VI

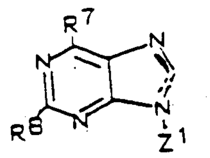

VI

ist, in der $Z^1$ Trialkylsilyl, $R^7$ Halogen und $R^8$ trialkylsilyliertes Acylamino bedeuten.

**5.** Verfahren gemäß einem oder mehreren der Ansprüche 1-3, dadurch gekennzeichnet, daß das eingesetzte Purinderivat eine Verbindung der Formel VII ist

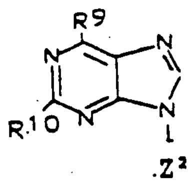

VII

in der $Z^2$ Acyl, $R^9$ Halogen und $R^{10}$ Acylamido bedeuten und nach der Reaktion gegebenenfalls der Substituent $R^9$ durch Hydrogenolyse in H umgewandelt wird und/oder der Substituent $R^{10}$ durch Ammonolyse, Aminolyse oder Hydrolyse in $NH_2$ umgewandelt wird.

**6.** Verbindungen der Formel II

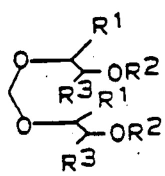

II

wobei die Substituenten $R^1$-$R^3$ die in Anspruch 1 genannte Bedeutung haben.

**7.** Verfahren zur Herstellung von Verbindungen der Formel II gemäß Anspruch 6, dadurch gekennzeichnet, daß Verbindungen der Formel I

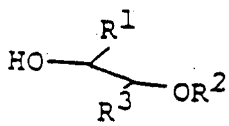

I

wobei $R^1$-$R^3$ die in Anspruch 1 genannten Bedeutungen haben, mit Paraformaldehyd, Formaldehyddimethylacetal oder Formaldehyddiethylacetal in Gegenwart eines Katalysators umgesetzt werden.

**8.** Verfahren zur Herstellung von mit einer

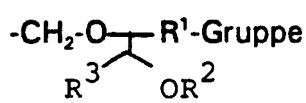

substituierten acyclischen Nukleosiden, dadurch gekennzeichnet, daß eine Verbindung der Formel II, gemäß Anspruch 6 mit einem geeigneten stickstoffhaltigen heterocyclischen System umgesetzt wird, wobei die Substituenten $R^1$-$R^3$ die in Anspruch 1 genannten Bedeutungen haben.

**9.** Verbindungen der Formel

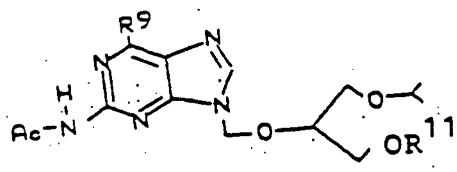

in der $R^9$ Halogen oder Wasserstoff und $R^{11}$ Isopropyl oder Benzyl bedeutet.

**Patentansprüche für folgende Vertragsstaaten: ES, GR**

**1.** Verfahren zur Herstellung von substituierten acyclischen Nukleosiden, dadurch gekennzeichnet, daß ein Alkohol der Formel I

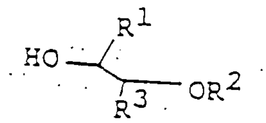

I

zu einem Formaldehydacetal der Formel II

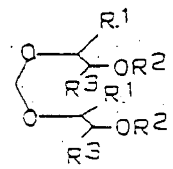

II

wobei

$R^1$ Wasserstoff, Alkyl, gegebenenfalls einfach oder mehrfach substituiert mit Halogen, Azid, Alkoxy-, Alkenyloxy-, Alkinyloxy-, Alkylthio-, Alkenylthio-, Alkinylthio-, Dialkylamino-, Dialkenylamino-, Dialkinylamino-, Benzyloxy-, Benzylthio-, Dibenzylamino-, oder Phthalimidogruppen und/oder mit - $P(O)(OR^4)(OR^5)$-, $-P(R^6)(O)(OR^5)$-, $-O-CH_2-P(O)(OR^4)(OR^5)$- oder $-O-CH_2P(R^6)(O)(OR^5)$-Resten, wobei $R^4$, $R^5$ und $R^6$ jeweils unabhängig voneinander $C_1$-$C_6$-Alkyl sein können, bedeutet;

$R^2$ Alkyl, Benzyl oder ein $-CH_2-P(O)(OR^4)(OR^5)$- oder $-CH_2-P(R^6)(O)(OR^5)$-Rest, wobei $R^4$, $R^5$ und $R^6$ jeweils unabhängig voneinander $C_1$-$C_6$-Alkyl sein können, bedeutet

$R^3$ Wasserstoff, Alkyl, gegebenenfalls einfach oder mehrfach substituiert mit Halogen, Azid, Alkoxy-, Alkenyloxy-, Alkinyloxy-, Alkylthio-, Alkenylthio-, Alkinylthio-, Dialkylamino-, Dialkenylamino-, Dialkinylamino-, Benzyloxy-, Benzylthio-, Dibenzylamino- oder Phthalimidogruppen und/oder mit $-P(O)(OR^4)(OR^5)$-, $-P(R^6)(O)(OR^5)$-, $-O-CH_2-P(O)(OR^4)(OR^5)$- oder $-O-CH_2-P(R^6)(O)(OR^5)$-Resten,

wobei $R^4$, $R^5$ und $R^6$ jeweils unabhängig voneinander $C_1$-$C_6$-Alkyl sein können, bedeutet, umgesetzt wird und zur Einführung der Gruppe

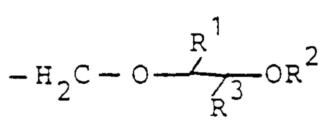

ein geeignet substituiertes stickstoffhaltiges heterocyclisches System mit einer Verbindung der Formel II zur Reaktion gebracht wird.

**2.** Verfahren gemäß Anspruch 1, dadurch gekennzeichnet, daß das stickstoffhaltige heterocyclische

System ausgewählt ist aus der folgenden Gruppe von Verbindungen:

Purine wie Purin, Adenin, 2-Chlor-6-aminopurin, Hypoxanthin, 6-Thiopurin, Xanthin, Guanin, 2-Amino-6-mercaptopurin, 2,6-Diaminopurin, 2-Aminopurin, 2,6-Diahlogenpurin; Azapurine wie 8-Azapurin, 8-Azaadenin, 8-Azaguanin; Deazapurine wie 1 -Deazapurine, 3-Deazapurine, 7-Deazapurine, 9-Deazapurine; Benzimidazole; Indole; Pyrimidine wie Cytosin, 5-Halogencytosine; 4-Amino-2-mercaptopyrimidin, Uracil, 5-Halogenuracile, 4-Hydroxy-2-mercaptopyrimidin, Thymin, 4-Hydroxy-2-mercapto-5-methyl-pyrimidin, 5-(2-Bromvinyl)uracil, 6-substituierte Pyrimidine wie 6-Phenylthiothymin; 2-Hydroxypyridine, 4-Hydroxypyridine; 1,2,3-Triazole, 1,2,4-Triazole, Tetrazole; Imidazole; Pyrrole.

**3.** Verfahren gemäß den Ansprüchen 1 oder 2, dadurch gekennzeichnet, daß das stickstoffhaltige heterocyclische System ein Purinderivat ist.

**4.** Verfahren gemäß einem oder mehreren der Ansprüche 1-3, dadurch gekennzeichnet, daß das eingesetzte Purinderivat eine Verbindung der Formel VI

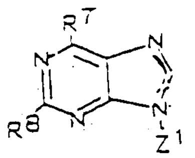

VI

ist, in der $Z^1$ Trialkylsilyl, $R^7$ Halogen und $R^8$ trialkylsilyliertes Acylamido bedeuten.

**5.** Verfahren gemäß einem oder mehreren der Ansprüche 1-3, dadurch gekennzeichnet, daß das eingesetzte Purinderivat eine Verbindung der Formel VII ist

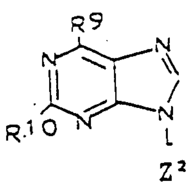

VII

in der $Z^2$ Acyl, $R^9$ Halogen und $R^{10}$ Acylamido bedeuten und nach der Reaktion gegebenenfalls der Substituent $R^9$ durch Hydrogenolyse in H umgewandelt wird und/oder der Substituent $R^{10}$ durch Ammonolyse, Aminolyse oder Hydrolyse in $NH_2$ umgewandelt wird.

**6.** Verfahren zur Herstellung von Verbindungen der Formel II, wobei die Substituenten $R^1$-$R^3$ die in Anspruch 1 genannten Bedeutung haben.

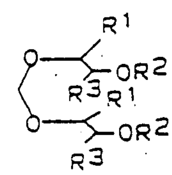

II

dadurch gekennzeichnet, daß Verbindungen der Formel I,

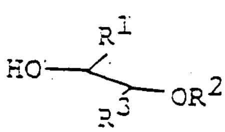

I

wobei $R^1$-$R^3$ die in Anspruch 1 genannten Bedeutungen haben, mit Paraformaldehyd Formaldehydimethylacetal oder Formaldehyddiethylacetal in Gegenwart eines Katalysators umgesetzt werden.

**7.** Verfahren zur Herstellung von mit einer

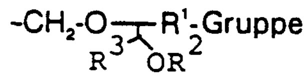

Gruppe

substituierten acyclischen Nukleosiden, dadurch gekennzeichnet, daß eine Verbindung der Formel II, gemäß Anspruch 6 mit einem geeigneten stickstoffhaltigen heterocyclischen System umgesetzt wird, wobei die Substituenten $R^1$-$R^3$ die in Anspruch 1 genannten Bedeutungen haben.

**8.** Verfahren gemäß Anspruch 7, dadurch gekennzeichnet, daß die Verbindung

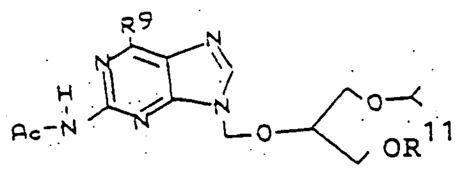

in der $R^9$ Halogen oder Wasserstoff und $R^{11}$ Isopropyl oder Benzyl bedeutet, hergestellt wird.